# FASCICULE DE BREVET EUROPEEN

(11) **EP 3 212 123 B1**
(45) Date de publication et mention de la délivrance du brevet: **20.07.2022**
(21) Numéro de dépôt: 15798517.7
(22) Date de dépôt: 28.10.2015
(51) Int. Cl.: A61D 19/02, A61B 90/00

(54) **APPAREIL POUR LA PÉNÉTRATION VAGINALE D'ANIMAUX COMPORTANT UN SYSTÈME DE VISION, NOTAMMENT POUR LOCALISER LE COL DE L'UTÉRUS**
VORRICHTUNG ZUR VAGINALEN PENETRATION VON TIEREN MIT EINEM BETRACHTUNGSSYSTEM, INSBESONDERE ZUM LOKALISIEREN DER ZERVIX DES UTERUS
APPLIANCE FOR VAGINAL PENETRATION OF ANIMALS, COMPRISING A VIEWING SYSTEM, ESPECIALLY FOR LOCATING THE CERVIX OF THE UTERUS

(30) Priorité: 28.10.2014 FR 1460352
(43) Date de publication de la demande: 06.09.2017
(73) Titulaire: IMV Technologies, 61300 Saint-Ouen-Sur-Iton (FR)
(72) Inventeur: BISCAY, Jean Arnaud, F-64120 Saint Palais (FR); ROSA, Virginie, F-64200 Biarritz (FR); DE RIBEROLLES, Esclarmonde, F-64510 Meillon (FR)
(74) Mandataire: Santarelli
(86) Numéro de dépôt international: PCT/FR2015/052909
(87) Numéro de publication internationale: WO 2016/066962

(56) Documents cités:
- EP-A2- 2 529 695
- WO-A1-02/05727
- WO-A1-97/14365
- FR-A1- 2 908 038
- JP-A- H03 275 053
- JP-A- 2010 213 948
- US-A- 2 942 603
- US-A1- 2009 270 897

## Description

Le présent mémoire concerne un appareil d'aide à, ou pour, la pénétration vaginale, ou adaptateur d'aide à, ou pour, la pénétration vaginale, pour des animaux, permettant d'effectuer différentes opérations, ainsi que des procédés d'utilisation de cet appareil.

Afin de réaliser une insémination artificielle d'animaux comme des ovins, des caprins, des porcins ou des bovins, un appareil connu, présenté notamment par le document FR-A1-2690072, comporte un pistolet allongé disposant d'une pointe avant permettant à l'opérateur de pénétrer dans le vagin pour atteindre le col de l'utérus, et d'injecter dans cet utérus au plus près des trompes la semence liquide contenue dans une paillette ou dans une capsule.

Toutefois pour certains animaux, en particulier pour les bovins comme les génisses n'ayant jamais vêlé, le passage au travers du vagin est délicat à réaliser car il comporte de nombreux plis resserrés qui sont difficiles à passer, et l'entrée du col de l'utérus est difficilement localisable, ainsi que franchissable car très étroite.

Pendant cette opération plus ou moins longue à réaliser en fonction de l'habitude de l'utilisateur et de la spécificité de l'animal, ce dernier peut s'énerver, notamment par la piqûre de la pointe du pistolet sur des tissus intérieurs sensibles, ce qui rend l'opération plus délicate et inconfortable, avec des contraintes pour l'éleveur ainsi qu'un risque d'erreur plus important.

Il y a alors plus de chances de réaliser une injection de semence sans résultat, ce qui occasionne du temps perdu, et gaspille des semences présentant un certain coût.

Par ailleurs une récente réglementation européenne autorise les éleveurs à réaliser les inséminations par eux-mêmes, sans passer par un organisme spécialisé, ce qui accroit l'intérêt pour des appareils simples et efficaces permettant de réaliser ces opérations avec un bon niveau de fiabilité, de confort et de sécurité, par un personnel qui pratique ces opérations de manière occasionnelle.

Des dispositifs d'insémination artificielle pour animaux comportant des moyens d'observation sont connus des documents EP2529695, JP2010213948, WO02/05727 et WO97/14365.

La présente invention a notamment pour but d'éviter ces inconvénients de la technique antérieure.

Elle propose à cet effet un ensemble de travail tel que défini dans la revendication 1. Des modes de réalisation préférés sont spécifiés dans les revendications dépendantes. Cet ensemble comporte un appareil pour la pénétration vaginale comprenant un manche prévu pour recevoir un outil de travail étant un pistolet d'insémination traversant le manche, l'appareil comporte un tube de guidage prolongeant le manche pour former un ensemble compact, le tube de guidage recevant à l'intérieur la pointe avant de l'outil de travail ainsi qu'un moyen de vision vidéo tel qu'un tube de type endoscope comportant un éclairage à son extrémité avant, l'appareil pour la pénétration vaginale comprenant une cellule électronique recevant les images vidéo et disposant de moyens de transmission des images vers un écran déporté.

L'ensemble de travail peut comporter aussi une seringue d'injection d'un antibiotique ou tout autre type de pistolet ou de seringue pour l'injection par pénétration vaginale d'une substance quelconque, par exemple de la semence, de l'antibiotique, ou tout autre produit.

Un avantage de cet appareil pour la pénétration vaginale est que grâce à la vision sur l'écran déporté qui n'est pas soumis aux mouvements de l'animal, permettant d'obtenir une image nette et stable, on peut manipuler aisément le manche compact qui offre ainsi une bonne prise en main, afin de faciliter l'insertion de l'extrémité du tube de guidage entre des plis ou des zones étroites, pour positionner notamment la pointe du pistolet à l'endroit précis assurant les meilleurs résultats de l'opération.

Un autre avantage de cet appareil est de limiter ou d'éviter des lésions qui peuvent être provoquées par la finesse de l'outil existant, notamment le pistolet d'insémination. Ceci est d'autant plus important lors de manipulations par un utilisateur occasionnel.

De plus l'écran déporté ne risque pas d'être souillé par des déjections de l'animal. L'opérateur peut ainsi travailler de manière détendue et rapide, sans être trop gêné par des mouvements de la queue de l'animal, et sans lui occasionner de stress important. L'utilisateur peut ainsi manipuler d'une seule main l'ensemble de l'appareil. La conception de cet appareil assure également le maintien de l'accessoire spécifique à l'usage, notamment le pistolet d'insémination, qui ainsi ne tombe pas par terre, en assurant l'hygiène et le confort d'usage.

Le système pour la pénétration, l'écran déporté et l'accessoire spécifique à l'usage sont ainsi manipulés par l'opérateur d'un seul bras, qui peut être le bras droit ou gauche. L'outil est ambidextre.

L'appareil pour la pénétration vaginale selon l'invention peut comporter de plus une ou plusieurs des caractéristiques suivantes, qui peuvent être combinées entre elles.

Selon l'invention, la cellule électronique est comprise dans le manche.

Avantageusement, les moyens de transmission des images comportent une prise pour une liaison filaire, ou un moyen de télécommunication sans fil. On peut ainsi facilement relier l'appareil à un écran extérieur.

En particulier, le moyen, tel qu'un tube, de vision peut comporter un système vidéo de type vidéoscopie ou fibroscopie.

Avantageusement, le moyen de vision peut être de type caméra, miniature, le cas échéant une telle caméra est fixée sur un support longiligne fixé, directement ou indirectement, sur le manche et dans le tube de guidage, avantageusement de manière à obtenir une focalisation de l'image sur l'axe de la pointe avant de l'outil de travail, à une distance de travail souhaitée en avant du tube de guidage, présentant par exemple une inclinaison de 3° (degrés) par rapport à l'axe de l'appareil d'insémination artificielle afin d'assurer une vision centrale et de donner l'impression à l'utilisateur que la caméra est dans l'axe de travail. Le mouvement semblera ainsi plus naturel à l'utilisateur.

Cet angle est défini par rapport à la distance du moyen de vision avec l'extrémité avant du tube de guidage, et dans le même temps afin que le centre de vision coïncide avec le centre du tube de guidage.

La caméra peut être fixée sur le support longiligne par l'intermédiaire de deux demi-coques destinées à coopérer entre elles pour être fixées entre elles en se refermant sur le support longiligne de manière à en être solidaires.

Avantageusement, la caméra comprend la cellule électronique.

Avantageusement, la caméra comprend les moyens de transmission de l'image.

Avantageusement, l'outil de travail présente des graduations de sorte à permettre à un utilisateur de connaître la mesure dans laquelle est avancée la pointe avant de l'outil de travail par rapport au manche de l'appareil pour la pénétration. De plus la graduation permet lors de l'acte d'insémination qui se réalise d'une main de garder centimètre après centimètre l'avancée du pistolet d'insémination dans le col de l'utérus sans risques pour l'animal. Si nécessaire en cas d'urgence cette même graduation permet de retirer très rapidement le pistolet du col de l'utérus afin de ne pas blesser l'animal. Grâce à cette graduation la pénétration dans le col se fait graduellement dans une parfaite maîtrise du geste.

Selon l'invention, l'outil de travail est un pistolet d'insémination, le pistolet d'insémination peut être est- compris dans un système comprenant une rallonge de pistolet à laquelle il est fixé, la rallonge pistolet étant conformée pour coulisser dans le manche selon l'axe du manche, sur laquelle est attachée une aiguille d'injection ou canule du pistolet comprenant une paillette contenant la semence à déverser. Bien entendu le pistolet d'insémination peut être remplacé par une seringue d'injection d'un antibiotique ou tout autre dispositif d'injection comportant une canule, la rallonge pistolet étant alors adaptée pour fixer cette une canule de la seringue dans laquelle circule un piston de la seringue.

Avantageusement, la rallonge pistolet est conformée pour que puisse y coulisser, par exemple à l'intérieur de la rallonge et dans l'axe de la rallonge, un bouton-poussoir, ou piston, du pistolet d'insémination.

Avantageusement, le bouton-poussoir est conformé pour actionner une tige-piston du pistolet d'insémination dont l'extrémité avant est prévue pour se déplacer dans la paillette disposée dans l'aiguille d'injection et à l'avant de la canule pour provoquer le déversage de la semence hors de la paillette et de l'aiguille d'injection du pistolet d'insémination.

Plus précisément et à titre d'exemple, concernant le pistolet d'insémination, le pistolet d'insémination comporte un corps tubulaire rigide, ou aiguille d'injection, pour recevoir la paillette remplie de semence, et comporte une tige d'entraînement, ou tige-piston, du bouchon de la paillette, montée coulissante dans le corps tubulaire rigide. Avant introduction de la paillette dans le corps tubulaire rigide, la tige est sortie ou reculée au maximum hors du corps tubulaire rigide du côté de l'extrémité proximale (vers l'arrière), c'est-à-dire du côté de l'extrémité qui est manipulée par l'inséminateur pendant l'opération, puis la paillette est introduite dans le corps tubulaire rigide à son extrémité distale (extrémité située du côté opposé à l'extrémité proximale, c'est-à-dire vers l'avant), la paillette étant introduite avec l'extrémité du tube la plus proche du bouchon la première. La paillette s'enfonce dans le corps tubulaire rigide jusqu'à ce que l'extrémité du tube rencontre un épaulement formant butée d'enfoncement. La paillette est alors en place dans le corps tubulaire rigide. L'extrémité du tube de la paillette la plus éloignée du bouchon ainsi qu'une certaine longueur du tube à partir de cette extrémité restent à l'extérieur du corps tubulaire rigide, c'est-à- dire qu'une certaine partie de la paillette saille au-delà de l'extrémité distale du corps tubulaire rigide du pistolet. Une gaine sanitaire comportant un tube dont le diamètre interne est tel que le corps tubulaire rigide du pistolet puisse y être introduit est alors mise en place. À une extrémité (extrémité proximale) le tube de la gaine sanitaire est ouvert et à l'autre extrémité (extrémité distale) le tube de la gaine sanitaire comporte un bord retourné formant un ourlet du côté interne. À l'intérieur de la gaine est disposé un manchon coulissant. Le corps tubulaire rigide du pistolet, dans lequel a été préalablement mise en place la paillette, est introduit dans la gaine par son extrémité ouverte avec la paillette la première, la paillette s'introduit dans le manchon coulissant et entraîne celui-ci. L'introduction dans la gaine se termine lorsque le manchon coulissant et la paillette viennent en butée contre le bord retourné formant ourlet situé à l'extrémité distale de la gaine (extrémité qui est du côté opposé à l'extrémité ouverte du tube de la gaine). La gaine est alors fixée au corps tubulaire rigide du pistolet, en général au voisinage de l'extrémité proximale de la gaine (extrémité ouverte du tube) par exemple avec une bague appropriée. Le tube de la paillette est ainsi immobilisé par rapport à l'ensemble formé par le corps tubulaire du pistolet et par la gaine sanitaire fixée à celui-ci. Lorsque l'ensemble formé par la gaine sanitaire et le pistolet est en place, la tige-piston est utilisée pour faire coulisser le bouchon de la paillette afin d'éjecter, ou déverser, la semence hors du tube de la paillette et hors du tube de la gaine par l'orifice entouré par le rebord formant ourlet. Le rôle du manchon coulissant est de fournir une étanchéité aux liquides entre le tube de la paillette et le tube de la gaine pour que la semence soit bien éjectée hors de la gaine (et non perdue entre le tube de la paillette et le tube de la gaine).

Avantageusement, la rallonge pistolet porte les graduations de mesure de l'avancée de l'aiguille d'injection du pistolet d'insémination, de manière à connaître dans le même temps l'avancée de la canule dans l'utérus de l'animal.

Avantageusement, les graduations sont gravées sur la rallonge pistolet et coopèrent avec des éléments montés dans le manche, tels que des billes montées sur ressort dans le manche, pour donner un effet de cran au franchissement de chaque graduation, ainsi l'utilisateur « sent » chaque graduation au fur et à mesure qu'elles coopèrent avec les éléments pour l'effet de cran, lui permettant d'être ainsi plus précis dans sa manipulation de la rallonge du pistolet et donc dans l'avancée du pistolet dans l'utérus.

Bien entendu, les éléments pour l'effet de cran en coopération avec les graduations gravées dans la rallonge pistolet permettent un retour en arrière de la rallonge pistolet pour rentrer le pistolet dans le tube de guidage de l'appareil pour la pénétration vaginale. Avantageusement, ils permettent à la rallonge pistolet de revenir sans intervention de l'utilisation lorsque l'animal bouge ou contracte ses muscles internes au voisinage du pistolet et plus précisément de l'extrémité avant de la canule du pistolet afin de préserver le bien-être de l'animal, tout en empêchant un retour inopiné (non désiré) de la rallonge pistolet sans mouvement ou contraction de l'animal ni intervention de l'utilisateur. Avantageusement, et pour ce faire, les éléments pour l'effet de cran sont montés dans le manche à l'aide d'un mécanisme qui autorise le désengagement des éléments pour l'effet de cran hors des graduations pour une force orthogonale à l'axe de la rallonge pistolet comprise entre 4 Newtons et 8 Newtons, de préférence sensiblement 6 Newtons.

Il est entendu que ce crantage est réalisable pour une rallonge d'une seringue d'injection d'un antibiotique par exemple.

Avantageusement, le manche comprend au moins une ouverture du manche de préférence oblongue s'étendant selon l'axe du manche de manière à alléger le manche de l'appareil pour la pénétration vaginale afin de rendre son utilisation moins contraignante et améliorer la prise du manche, par exemple lorsqu'une main de l'utilisateur ne peut faire un tour complet du manche lors de la préhension de ce dernier, la préhension n'étant donc pas satisfaisante et entraînant un risque de mauvaise manipulation de l'appareil pour la pénétration vaginale lors des opérations d'insémination artificielle. D'autre part, l'ouverture du manche permet de faire coulisser le pistolet par l'intermédiaire d'un doigt de l'utilisateur, toute la manipulation de l'appareil pour la pénétration vaginale pouvant se faire d'une seule main de manière simple. Enfin l'ouverture du manche permet un meilleur nettoyage du manche après l'utilisation de l'appareil.

La cellule arrière de l'outil peut comporter une batterie intégrée dans le manche.

Avantageusement, le tube de guidage peut être réalisé dans une matière transparente facilement lavable. On vérifie ainsi aisément sa propreté.

Avantageusement, le tube de guidage, le tube de vision et un tube recevant la pointe avant de l'outil de travail disposé dedans, sont fixés sur le manche par un montage pouvant être de type baïonnette assurant une fiabilité de la liaison ainsi qu'un démontage rapide et un lavage aisé.

Avantageusement, le tube de guidage comporte à sa base du côté du manche, des ouvertures vers l'extérieur assurant un système de ventilation mécanique. On évacue ainsi facilement les sécrétions se trouvant dans l'utérus, qui viennent dans ce tube ainsi que l'air présent dans le vagin.

Avantageusement, l'ensemble formé par le manche et le tube de guidage, comporte une forme globalement cylindrique. Cette conception assure à l'utilisateur un confort lié à une facilité d'atteinte du col de l'utérus, et une impossibilité d'accroché par la queue de l'animal, notamment les vaches, ce qui évite tout risque de chute et de destruction du matériel par l'animal.

Avantageusement, le tube de guidage comporte une face avant qui est inclinée pour faciliter la pénétration.

Avantageusement, l'appareil pour la pénétration peut comporter un moyen de chauffage du pistolet, qui permet de maintenir en température une semence.

Avantageusement, le tube de vision comprend une extrémité avant coudée de manière à obtenir une focalisation de l'image sur l'axe de la pointe avant de l'outil de travail, à une distance de travail souhaitée en avant du tube de guidage.

Bien que ne faisant pas partie de l'invention, il est aussi décrit un procédé d'utilisation d'un appareil pour la pénétration vaginale comprenant l'une quelconque des caractéristiques précédentes, équipé d'un pistolet d'insémination, comportant une première étape en surveillant la vision en avant du tube de guidage, de traversée du vagin en inclinant le manche de l'appareil en fonction des obstacles, puis d'éclairage du fond de ce vagin pour localiser l'entrée du col de l'utérus, afin de disposer l'extrémité avant du tube de guidage devant ce col, puis une deuxième étape en maintenant le manche dans cette position, de poussée du pistolet d'insémination pour le faire entrer dans le col de l'utérus, et enfin de poussée de la semence dans cet utérus.

Bien que ne faisant pas partie de l'invention, il est aussi décrit un procédé d'utilisation d'un appareil pour la pénétration vaginale comprenant l'une quelconque des caractéristiques précédentes, équipé d'un outil de dépose de médicaments, notamment d'antibiotiques, comportant une première étape en surveillant la vision en avant du tube de guidage, de traversée du vagin en inclinant le manche de l'appareil en fonction des obstacles, puis d'éclairage du fond de ce vagin pour localiser l'entrée du col de l'utérus, afin de disposer l'extrémité avant du tube de guidage devant ce col, puis une deuxième étape en maintenant le manche dans cette position, d'injection des médicaments dans l'utérus.

Bien que ne faisant pas partie de l'invention, il est aussi décrit un procédé d'utilisation d'un appareil pour la pénétration vaginale comprenant l'une quelconque des caractéristiques précédentes, équipé d'un outil d'écoute des sons, comportant une première étape en surveillant la vision en avant du tube de guidage, de traversée du vagin en inclinant le manche de l'appareil en fonction des obstacles, puis d'éclairage du fond de ce vagin pour localiser l'entrée du col de l'utérus, afin de disposer l'extrémité avant du tube de guidage devant ce col, puis une deuxième étape en maintenant le manche dans cette position, d'écoute des sons émis au-delà du col.

Bien que ne faisant pas partie de l'invention, il est aussi décrit un procédé d'utilisation d'un appareil pour la pénétration vaginale comprenant l'une quelconque des caractéristiques précédentes, équipé d'un outil de photographie, comportant une première étape en surveillant la vision en avant du tube de guidage, de traversée du vagin en inclinant le manche de l'appareil en fonction des obstacles, puis d'éclairage du fond de ce vagin pour localiser l'entrée du col de l'utérus, afin de disposer l'extrémité avant du tube de guidage devant ce col, puis une deuxième étape en maintenant le manche dans cette position, de photographie de ce col. Des annotations et autres peuvent être reliés aux images récoltées.

Bien que ne faisant pas partie de l'invention, il est aussi décrit un procédé d'utilisation d'un appareil pour la pénétration vaginale comprenant l'une quelconque des caractéristiques précédentes, équipé d'un outil de prélèvement de substances internes, comportant une première étape en surveillant la vision en avant du tube de guidage, de traversée du vagin en inclinant le manche de l'appareil en fonction des obstacles, puis d'éclairage du fond de ce vagin pour localiser l'entrée du col de l'utérus, afin de disposer l'extrémité avant du tube de guidage devant ce col, puis une deuxième étape en maintenant le manche dans cette position, de prélèvement des substances.

L'invention sera mieux comprise et d'autres caractéristiques et avantages apparaîtront plus clairement à la lecture de la description ci-après donnée à titre d'exemple, en référence aux dessins annexés dans lesquels :
- les figures 1a et 1b sont des vues d'ensemble d'un appareil pour la pénétration selon l'invention, comprenant un pistolet d'insémination ;
- la figure 2 est une vue de détail du manche de cet appareil ;
- la figure 3 est une vue de détail de la bague intermédiaire ;
- la figure 4 est une vue de détail du tube de guidage ;
- la figure 5 est une vue de détail du tube du pistolet ; et
- la figure 6 est une vue de détail du tube de vision.

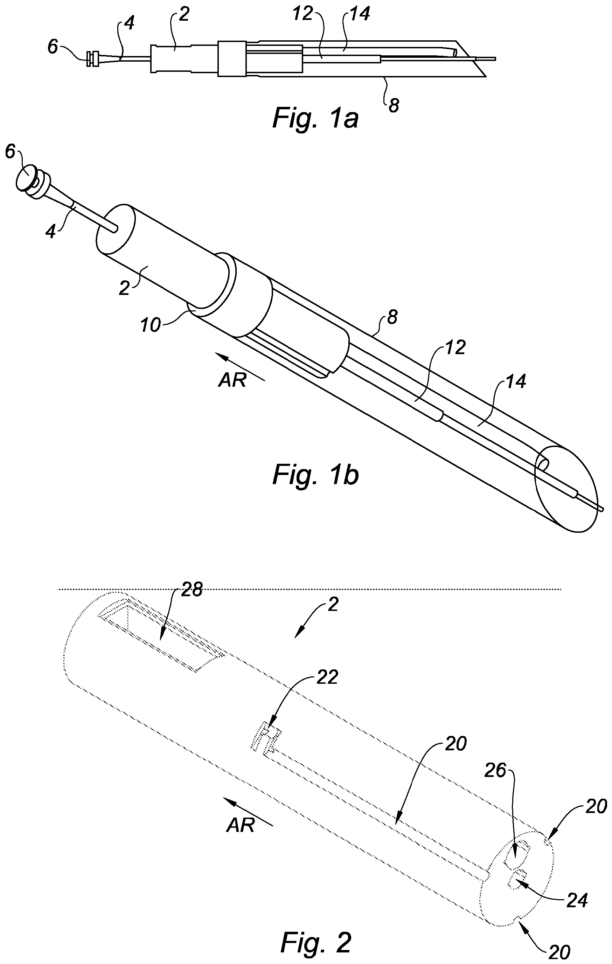

Les figures 1a et 1b présentent un appareil pour la pénétration formant un ensemble comprenant une forme allongée qui est sensiblement cylindrique, présentant vers l'arrière indiqué par la flèche « AR », un manche 2 recevant suivant son axe un pistolet d'injection 4 coulissant dedans, comportant une aiguille d'injection, ou tige du pistolet d'insémination contenant la paillette, se terminant à l'arrière par un bouton-poussoir 6, et à l'avant par une pointe.

Le manche se prolonge vers l'avant par un tube de guidage réalisé en matière plastique transparente 8, qui est fixé au milieu de ce manche par une bague intermédiaire de serrage 10. Le tube de guidage 8 comporte à l'intérieur un tube axial 12 entourant la pointe avant du pistolet, et à côté un tube d'endoscope 14 d'un système vidéo. Le diamètre de ce tube de guidage 8 est dans des dimensions qui respectent le bien-être de l'animal, et facilitent la pénétration, et surtout présentant un diamètre moindre que ceux déjà connus de manière à convenir à la majorité des animaux que l'on souhaite inséminer. À titre d'exemple, dans le cas des vaches, le diamètre est de préférence compris entre 30 millimètres et 43 millimètres, le diamètre est de préférence de sensiblement 36 millimètres et peut ainsi convenir à la quasi-totalité des bovins tout en respectant leur bien-être et afin d'améliorer les conditions d'insémination, notamment des génisses, là ou les appareils pour la pénétration vaginale connus pouvaient avoir un diamètre de 4 centimètres ou 5 centimètres. Dans le cas d'une application pour les ovins, le diamètre pourrait être encore réduit.

La figure 2 présente le manche 2 comprenant un contour extérieur cylindrique, présentant trois rainures longitudinales 20 partant de l'avant, qui se terminent chacune sensiblement au milieu de ce manche par une rainure 22 formant un petit créneau comprenant une première partie transversale, une partie longitudinale courte, et une deuxième partie transversale parallèle à la première.

Le manche 2 présente un perçage axial 24 qui le traverse de part en part. La partie avant du perçage axial 24 comporte sur une petite longueur un diamètre plus important comprenant deux rainures longitudinales qui se terminent chacune à l'arrière par une courte rainure transversale suivant un arc de cercle, afin de recevoir de manière ajustée la partie arrière du tube du pistolet, et de la fixer par un montage du type baïonnette.

Le manche 2 comporte un deuxième perçage longitudinal 26 décalé par rapport à l'axe, qui part de l'avant, et débouche vers l'arrière dans une cavité allongée 28 ouverte sur un côté de ce manche, prévue pour contenir une cellule arrière de l'endoscope recevant les images venant de l'extrémité du tube de vision 14.

La cellule arrière de l'appareil peut recevoir l'énergie électrique par un câble extérieur, relié par exemple à une tablette ou un smartphone, ou téléphone intelligent, effectuant l'affichage. En variante une batterie peut être intégrée dans le manche 2 pour alimenter cette cellule arrière.

La cavité 28 est prévue pour recevoir un couvercle non représenté, qui est ajusté sur la surface cylindrique extérieure, afin de la fermer pour protéger à l'intérieur la cellule du système vidéo tout en présentant un contour extérieur du manche 2 qui est lisse.

Comme pour le perçage axial 24, le perçage déporté 26 comporte sur une partie avant deux rainures longitudinales qui se terminent chacune à l'arrière par une rainure transversale, pour recevoir le tube d'endoscope 14 et le fixer par un montage du type baïonnette.

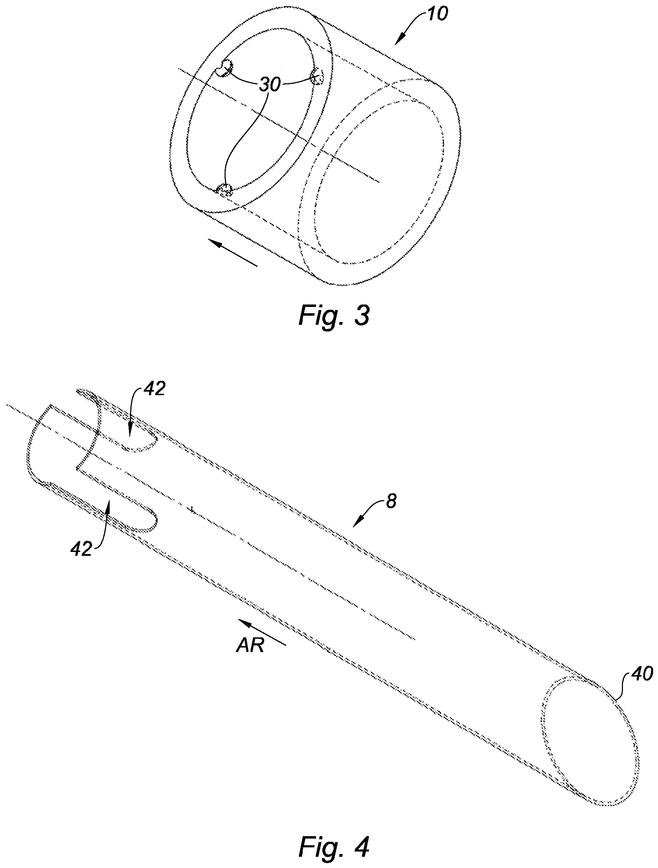

La figure 3 présente la bague intermédiaire cylindrique 10, prévue pour recevoir le tube de guidage 8 qui est collé dessus.

La bague intermédiaire 10 comprend un diamètre intérieur s'ajustant sur le contour extérieur du manche 2, qui présente trois ergots 30 dépassant vers l'intérieur, prévus pour rentrer chacun dans une rainure extérieure longitudinale 20 de ce manche en partant de l'avant.

Une fois dans la position arrière, l'ensemble formé par le tube de guidage 8 et sa bague intermédiaire 10 fixée dedans, est manœuvré pour faire circuler chaque ergot 30 dans le créneau 22, en le tournant, le reculant vers l'arrière, puis le retournant dans le sens inverse. On réalise ainsi un montage rapide et sécurisé du tube de guidage 8 sur le manche 2, qui assure un positionnement précis.

La figure 4 présente le tube de guidage 8 réalisé en matière plastique transparente comme un plexiglas, comprenant une face avant 40 inclinée facilitant l'avancée vers le col utérin, et une extrémité arrière ajustée dans la bague intermédiaire 10, qui est serrée et collée dedans.

La partie arrière du tube de guidage 8 comporte deux fentes longitudinales opposées 42 qui débouchent vers l'arrière. La partie avant de chaque fente longitudinale 42 vient en avant de la bague intermédiaire 10, de manière à former deux ouvertures opposées quand le tube de guidage 8 est monté sur le manche 2.

Les ouvertures des deux fentes 42 permettent quand le tube de guidage est introduit dans l'animal, de réaliser un passage d'air qui communique avec les volumes intérieurs de l'animal, et d'évacuer des sécrétions se trouvant dans le vagin et venant dans ce tube lors de son introduction. En inclinant le manche vers le bas on permet à ces sécrétions de glisser le long du tube, et de sortir par une des fentes 42.

La matière transparente du tube de guidage 8 permet de suivre son état de propreté intérieure pendant les opérations, et après lors de son lavage et de sa désinfection.

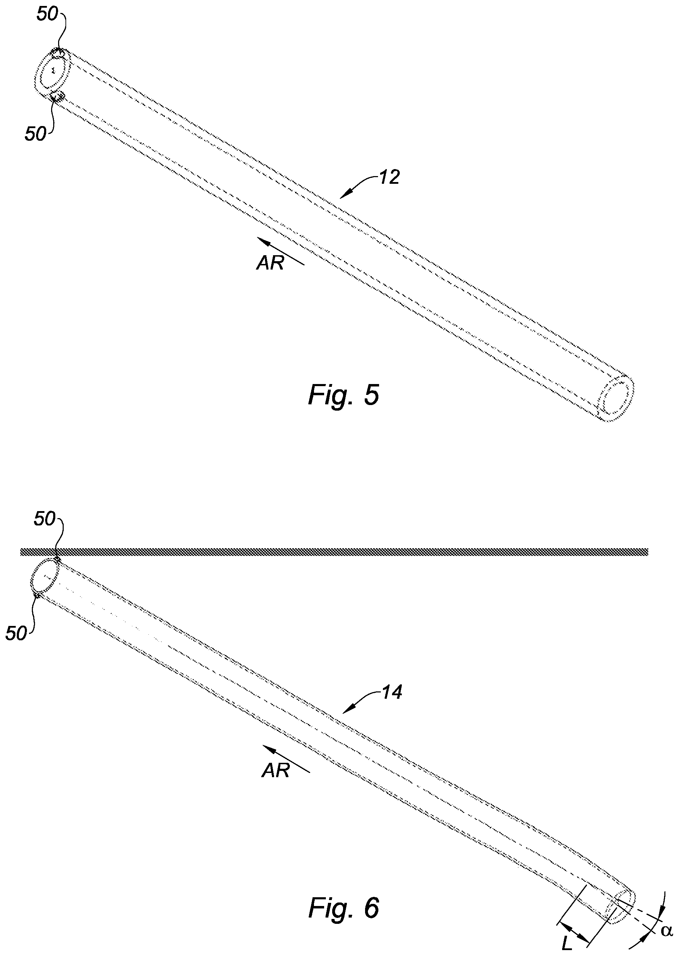

La figure 5 présente le tube du pistolet 12 comprenant un diamètre constant, sa partie arrière venant s'ajuster dans la partie avant du perçage axial 24 du manche 2. L'extrémité arrière du tube du pistolet 12 présente deux ergots 50 diamétralement opposés tournés vers l'extérieur, qui s'ajustent chacun dans une fente longitudinale de ce perçage axial 24.

Une fois poussé dans sa position arrière, on réalise une rotation du tube du pistolet 12 afin de disposer les ergots 50 dans les rainures transversales du manche 2, pour réaliser une fixation du type baïonnette, qui est simple, rapide et efficace.

L'extrémité avant du tube du pistolet 12 arrive sensiblement au milieu de la longueur du tube de guidage 8 dépassant en avant du manche 2.

La figure 6 présente le tube de vision 14 comprenant comme pour le tube du pistolet 12, un diamètre constant, et une partie arrière s'ajustant dans la partie avant du perçage déporté 26 du manche 2, qui est équipée à son extrémité de deux ergots 50 afin de réaliser un montage du type baïonnette sur ce manche.

L'extrémité avant du tube de vision 14 réalisant en même temps un éclairage, donné notamment par des cellules du type « LED », ainsi qu'une vision vers l'avant, s'arrête un peu en retrait de la face avant 40 du tube de guidage 8 qui protège ainsi cette extrémité.

La partie avant du tube de vision 14 est sur une petite longueur L, coudée suivant un angle α de manière à obtenir une focalisation de l'image sur l'axe de l'appareil à une distance de travail souhaitée en avant de ce tube, qui se trouve sensiblement à la pointe de l'aiguille d'injection quand elle est avancée. On peut ainsi surveiller la dépose de la semence par la pointe de l'aiguille.

D'une manière générale pour l'usage d'un autre outil de travail dans l'appareil pour la pénétration, on peut de la même manière surveiller le travail de la pointe.

Le tube de vision 14 peut comporter différents systèmes de vision. On peut utiliser notamment un système de fibroscopie réalisant la transmission d'images par fibres optiques qui envoient la lumière vers l'avant, et retournent les images à la cellule arrière située dans la cavité 28 du manche 2. En variante on peut utiliser un système vidéo, l'extrémité du tube de vision 14 comportant un moyen d'éclairage, ainsi qu'un capteur photosensible, par exemple du type « CCD ».

La focalisation du moyen de vision est faite sensiblement sur la pointe du pistolet 4 qui se trouve dans sa position avant, pour pouvoir suivre avec précision la dépose de la semence qui se trouve à cette pointe.

Le manche 2 peut comporter un bouton de mise en marche du système vidéo, qui n'est pas représenté sur les figures. La cellule arrière du système vidéo communique avec le système d'affichage qui est dissocié de l'appareil pour la pénétration, par une liaison filaire, en utilisant par exemple une prise du type « USB » se trouvant sur le manche, ou par une liaison sans fil, en utilisant par exemple une communication radio du type « Bluetooth ».

On obtient ainsi avec une liaison radio ou filaire « USB », un système adapté à de nombreux outils existant sur le marché. Une application logicielle programmée peut être installée par téléchargement sur la majorité des appareils du marché. Cette application assure la communication entre l'appareil déporté et le système radio. L'application permet à l'utilisateur d'entrer un nombre évolutif d'informations sur l'action réalisée par l'utilisateur et l'animal concerné. Les données enregistrées par cette application peuvent instantanément ou dans un deuxième temps, être synchronisées avec une base de donnée. Des prestations de services facilitant la vie et le bien-être de l'utilisateur et de l'animal pourront être définies et développées au fur et à mesure des usages.

Dans le cas d'une liaison sans fil, le manche 2 contient l'électronique de contrôle des moyens d'éclairage et de vision, ainsi qu'une source d'alimentation électrique autonome.

Le système d'affichage peut en particulier utiliser un téléphone portable ou une tablette. Il peut utiliser notamment un appareil du type « Smartphone » disposé par exemple dans une housse étanche comportant des moyens de fixation, en particulier une bande comprenant une fixation rapide du type « Velcro » pour le fixer au bras de l'utilisateur.

Le système d'affichage peut aussi utiliser tout autre système de vision, notamment un système intégré dans des lunettes avec une communication radio du type « Bluetooth ».

De cette manière l'opérateur peut suivre sur un écran stable et propre l'avancée de l'extrémité avant de l'appareil, tout en guidant cette extrémité par des inclinaisons du manche afin de faciliter le passage entre les différents tissus des cavités intérieures qui se présentent.

Les images obtenues, photographies ou films, peuvent être stockées dans une mémoire puis transférées dans une base de données, ce qui permet de les ressortir par la suite pour suivre l'évolution de l'animal. On peut par exemple en référençant le type de paillette utilisé pour une insémination, stocker des données sur les opérations effectuées sur chaque animal.

Ces images peuvent aussi être transmises, notamment par Internet, à une personne extérieure comme un vétérinaire, pour réaliser un diagnostic, ou suivre l'état de santé des animaux.

Le mode de fonctionnement de l'appareil est le suivant. Le moyen de vision et l'écran déporté permettent de suivre la trajectoire dans le vagin de l'appareil pour la pénétration vaginale, puis d'éclairer le fond du vagin pour localiser l'entrée du col de l'utérus, afin de disposer l'extrémité avant du tube de guidage devant le col de l'utérus. Ensuite l'opérateur tenant d'une main le manche dans cette position, pousse le pistolet d'insémination (4) de cette même main chevauchant le manche et le pistolet d'insémination, la rallonge du pistolet d'insémination selon le cas, pour le faire entrer dans le col jusqu'au corps utérin, et enfin pousse le contenu de la paillette dans cet utérus toujours avec la même main chevauchant alors le manche et le bouton-poussoir, fonctionnant alors à la manière d'un piston.

En cours d'opération suivant l'état intérieur constaté par la vision, notamment la dilatation du col, la présence de glaires ou la propreté de ces glaires, l'opérateur peut décider ou pas d'effectuer l'injection de semences. Les chances de succès de l'insémination sont ainsi fortement assurées.

L'appareil pour la pénétration peut utiliser comme outil de travail différents types de pistolet d'insémination connus, ou des outils pour la dépose de différents produits, notamment des antibiotiques.

Des accessoires peuvent être proposés pour adapter l'appareil pour la pénétration aux différents usages. On peut en particulier prévoir dans le manche de l'appareil un système de préchauffage du pistolet, utilisant par exemple une résistance électrique, ou une substance chauffante ou isolante adaptée, afin d'éviter des écarts thermiques entre la décongélation de la semence et son insertion, qui diminuent son activité.

On peut utiliser comme outil de travail un outil d'écoute des sons au-delà du col de l'utérus, permettant en disposant l'extrémité avant du tube de guidage 8 devant ce col, d'écouter des sons émis au-delà.

On peut utiliser aussi un outil de photographie du col de l'utérus. On peut de plus utiliser un outil de prélèvement de substances, pour réaliser notamment un examen cytologique par prélèvement.

Après une opération les différents tubes de l'appareil pour la pénétration, qui sont tous fixés sur le manche par le montage rapide du type baïonnette, sont facilement déposés pour réaliser un lavage complet de l'appareil.

Il est à ajouter que l'appareil pour la pénétration vaginale peut bien entendu recevoir des outils déjà existants si cela est utile et que seules des modifications mineures pour l'adaptation de l'appareil sont à faire à cette fin et ce sans difficultés quelconques.

Dans une variante non illustrée, un mécanisme télescopique d'actionneurs permettant aussi de mettre en œuvre d'une seule main l'appareil pour la pénétration est décrit. Dans cette variante, une fois l'appareil pour la pénétration introduit jusqu'au col de l'utérus à l'aide d'un manche (2), le pistolet d'insémination, et plus précisément une rallonge du pistolet d'insémination, coulisse à l'intérieur du manche (2) et qu'enfin, de manière classique cette fois, le contenu de la paillette disposée à l'intérieur et à l'avant du pistolet est déversé au moyen du bouton-poussoir (6) fonctionnant de la même manière qu'un piston, c'est-à-dire coulissant à l'intérieur du corps du pistolet vers l'avant du pistolet pour pousser le contenu de la paillette dans l'utérus. De préférence, et de manière classique, le piston présente un diamètre inférieur au diamètre du pistolet de manière à coulisser sans heurts à l'intérieur même du pistolet et le piston permet alors de pousser un bouchon se trouvant dans la paillette qui lui-même pousse le contenu de la paillette, c'est-à-dire la semence, dans l'utérus sous l'action du piston. Le bouchon peut être d'une constitution classique comprenant par exemple trois strates successives à l'intérieur et le long de la paillette dont une première de bourre une seconde de gel et une troisième à nouveau de bourre. Le bouchon est initialement, avant toute opération visant au déversage de la semence, disposé en arrière de la paillette c'est-à-dire dans une partie de la paillette le plus proche du manche de l'appareil pour la pénétration. Le piston poussant le bouchon vers l'avant de la paillette, la bourre au contact du piston permet de pousser de manière optimale le gel et la troisième strate de bourre dans la paillette.

Bien entendu cette dernière variante non illustrée présentant un mécanisme télescopique est applicable pour tout type d'outil de travail, à titre d'exemples non limitatifs, un outil de dépose de médicaments, un outil d'écoute de sons, un outil de prise de photographies ou un outil de prélèvement de substances internes.

Le mécanisme télescopique comprend au moins le manche et la rallonge pistolet, et optionnellement le bouton-poussoir, qui est optionnel sur un outil d'écoute de sons par exemple.

Enfin, dans une variante non illustrée, le bouton-poussoir peut être actionné si nécessaire par une rallonge piston, qui est une rallonge du bouton-poussoir, coulissant à l'intérieur de la rallonge pistolet, cette rallonge piston, lorsqu'elle est utile, fait partie du mécanisme télescopique.

## Revendications

1. - Ensemble de travail, comportant :
- un appareil d'aide à la pénétration vaginale comprenant : un manche (2) ; un tube de guidage (8) prolongeant le manche (2) pour former un ensemble compact, ledit tube de guidage (8) présentant une extrémité avant configurée pour être disposée devant le col de l'utérus d'un animal ; et un tube de vision vidéo de type endoscopie (14), comportant un éclairage à son extrémité avant, le tube de vision vidéo étant reçu dans le tube de guidage (8) ; le manche (2) comprenant une cellule arrière de système vidéo disposant de moyens de transmission, vers un écran déporté, d'images venant de l'extrémité du tube de vision vidéo ; et
- un outil de travail reçu dans ledit appareil, traversant le manche, vis-à-vis duquel il est coulissant, grâce à quoi l'outil de travail est mobile entre une position où sa pointe avant est reçue à l'intérieur du tube de guidage (8) et une position où sa pointe avant est en avant du tube de guidage, ledit outil de travail étant un pistolet d'insémination (4) configuré pour pénétrer dans le vagin dudit animal, atteindre ledit col de l'utérus, en franchir l'entrée et injecter dans l'utérus dudit animal de la semence liquide contenue dans une paillette ou une capsule.

2. - Ensemble selon la revendication 1, **caractérisé en ce que** l'extrémité avant du tube de vision (14) est configurée de façon à réaliser en même temps un éclairage ainsi qu'une vision vers l'avant.

3. - Ensemble selon l'une quelconque des revendications précédentes, **caractérisé en ce que** le tube de vision (14) comporte un système de vidéo avec éclairage de type vidéoscopie ou fibroscopie.

4. - Ensemble selon l'une quelconque des revendications précédentes, **caractérisé en ce que** le tube de guidage (8) comporte à l'intérieur, à côté du tube de vision (14), un tube (12) configuré pour recevoir la pointe avant de l'outil de travail disposé dedans.

5. - Ensemble selon l'une quelconque des revendications précédentes, **caractérisé en ce que** les moyens de transmission d'images comportent une prise pour une liaison filaire.

6. - Ensemble selon la revendication 5, **caractérisé en ce que** ladite prise est du type « USB ».

7. - Ensemble selon l'une quelconque des revendications 1 à 4, **caractérisé en ce que** les moyens de transmission d'images comportent un moyen de télécommunication sans fil.

8. - Ensemble selon l'une quelconque des revendications précédentes, **caractérisé en ce que** la cellule arrière de l'appareil est configurée pour recevoir l'énergie électrique par un câble extérieur relié à une tablette ou un smartphone effectuant l'affichage.

9. - Ensemble selon l'une quelconque des revendications 1 à 7, **caractérisé en ce que** la cellule arrière de l'appareil comporte une batterie intégrée dans le manche (2).

10. - Ensemble selon l'une quelconque des revendications précédentes, **caractérisé en ce que** le tube de guidage (8) est réalisé dans une matière transparente facilement lavable.

11. - Ensemble selon l'une quelconque des revendications précédentes, **caractérisé en ce que** le tube de guidage (8), le moyen de vision (14), et un tube recevant la pointe avant de l'outil de travail (12) disposé dedans, sont fixés sur le manche (2) par un montage du type baïonnette.

12. - Ensemble selon l'une quelconque des revendications précédentes, **caractérisé en ce que** le tube de guidage (8) comporte à sa base du côté du manche (2), des ouvertures vers l'extérieur (42).

13. - Ensemble selon l'une quelconque des revendications précédentes, **caractérisé en ce que** l'ensemble compact formé par le manche (2) et le tube de guidage (8), comporte une forme globalement cylindrique.

14. - Ensemble selon l'une quelconque des revendications précédentes, **caractérisé en ce qu'**il comprend un mécanisme télescopique comprenant au moins le manche (2) et une rallonge pistolet.

15. - Ensemble selon l'une quelconque des revendications précédentes, **caractérisé en ce que** le tube de guidage (8) comporte une face avant (40) qui est inclinée.

16. - Ensemble selon l'une quelconque des revendications précédentes, **caractérisé en ce qu'**il comporte un moyen de chauffage du pistolet (4).

17. - Ensemble selon l'une quelconque des revendications précédentes, **caractérisé en ce que** le tube de vision (14) est orienté de manière à obtenir une focalisation de l'image sur l'axe de la pointe avant de l'outil de travail (4), à une distance de travail souhaitée en avant du tube de guidage (8).

18. - Ensemble selon l'une quelconque des revendications précédentes, **caractérisé en ce que** le tube de vision (14) comprend une extrémité avant coudée de manière à obtenir une focalisation de l'image sur l'axe de la pointe avant de l'outil de travail (4), à une distance de travail souhaitée en avant du tube de guidage (8).

19. - Ensemble selon l'une quelconque des revendications 1 à 18, **caractérisé en ce qu'**il comporte également un autre outil de travail (4) qui est un outil de dépose de médicaments.

20. - Ensemble selon l'une quelconque des revendications 1 à 18, **caractérisé en ce qu'**il comporte également un autre outil de travail (4) qui est un outil d'écoute de sons.

21. - Ensemble selon l'une quelconque des revendications 1 à 18, **caractérisé en ce qu'**il comporte également un autre outil de travail (4) qui est un outil de prise de photographies.

22. - Ensemble selon l'une quelconque des revendications 1 à 18, **caractérisé en ce qu'**il comporte également un autre outil de travail (4) qui est un outil de prélèvement de substances internes.

## Patentansprüche

1. Arbeitsanordnung, enthaltend:
- ein Gerät zur Unterstützung der Vaginalpenetration mit einem Schaft (2); einem Führungsrohr (8), das den Schaft (2) fortsetzt, um eine kompakte Anordnung zu bilden, wobei das Führungsrohr (8) ein vorderes Ende aufweist, das dazu ausgelegt ist, vor den Gebärmutterhals eines Tieres angeordnet zu werden; und einer endoskopischen Videosichtröhre (14), die an ihrem vorderen Ende eine Beleuchtung aufweist, wobei die Videosichtröhre in dem Führungsrohr (8) aufgenommen ist; wobei der Schaft (2) eine hintere Videosystemzelle aufweist, die über Mittel zum Übertragen von Bildern von dem Ende der Videosichtröhre an einen entfernt liegenden Bildschirm verfügt; und
- ein Arbeitswerkzeug, welches in dem Gerät aufgenommen ist, sich durch den Schaft hindurch erstreckt und zu diesem verschiebbar ist, wodurch das Arbeitswerkzeug zwischen einer Stellung, in welcher seine vordere Spitze im Inneren des Führungsrohrs (8) aufgenommen ist, und einer Stellung, in welcher seine vordere Spitze sich vor dem Führungsrohr befindet, beweglich ist, wobei das Arbeitswerkzeug eine Besamungspistole (4) ist, die dazu ausgelegt ist, in die Vagina des Tieres einzudringen, den Gebärmutterhals zu erreichen, dessen Eingang zu durchqueren und flüssigen Samen, der in einem Röhrchen oder einer Kapsel enthalten ist, in die Gebärmutter des Tieres zu injizieren.

2. Anordnung nach Anspruch 1,
**dadurch gekennzeichnet, dass**
das vordere Ende der Sichtröhre (14) dazu ausgelegt ist, gleichzeitig eine Beleuchtung und eine Sicht nach vorne zu ermöglichen.

3. Anordnung nach einem der vorhergehenden Ansprüche,
**dadurch gekennzeichnet, dass**
die Sichtröhre (14) ein videoskopisches oder endoskopisches Videosystem mit Beleuchtung enthält.

4. Anordnung nach einem der vorhergehenden Ansprüche,
**dadurch gekennzeichnet, dass**
das Führungsrohr (8) innen neben der Sichtröhre (14) eine Röhre (12) enthält, die dazu ausgelegt ist, die vordere Spitze des darin angeordneten Arbeitswerkzeugs aufzunehmen.

5. Anordnung nach einem der vorhergehenden Ansprüche,
**dadurch gekennzeichnet, dass**
die Mittel zur Bildübertragung einen Anschluss für eine Kabelverbindung enthalten.

6. Anordnung nach Anspruch 5,
**dadurch gekennzeichnet, dass**
der Anschluss ein USB-Anschluss ist.

7. Anordnung nach einem der Ansprüche 1 bis 4,
**dadurch gekennzeichnet, dass**
die Mittel zur Bildübertragung ein drahtloses Telekommunikationsmittel enthalten.

8. Anordnung nach einem der vorhergehenden Ansprüche,
**dadurch gekennzeichnet, dass**
die hintere Zelle des Geräts dazu ausgelegt ist, elektrische Energie über ein externes Kabel zu empfangen, das mit einem Tablet oder Smartphone verbunden ist, das die Anzeige durchführt.

9. Anordnung nach einem der Ansprüche 1 bis 7,
**dadurch gekennzeichnet, dass**
die hintere Zelle des Geräts eine in den Schaft (2) integrierte Batterie aufweist.

10. Anordnung nach einem der vorhergehenden Ansprüche,
**dadurch gekennzeichnet, dass**
das Führungsrohr (8) aus einem leicht abwaschbaren, transparenten Material hergestellt ist.

11. Anordnung nach einem der vorhergehenden Ansprüche,
**dadurch gekennzeichnet, dass**
das Führungsrohr (8), die Sichteinrichtung (14) und eine Röhre, welche die vordere Spitze des darin angeordneten Arbeitswerkzeugs (12) aufnimmt, mittels bajonettartiger Montage am Schaft (2) befestigt sind.

12. Anordnung nach einem der vorhergehenden Ansprüche,
**dadurch gekennzeichnet, dass**
das Führungsrohr (8) an seiner Basis auf der Seite des Schafts (2) nach außen gerichtete Öffnungen (42) aufweist.

13. Anordnung nach einem der vorhergehenden Ansprüche,
**dadurch gekennzeichnet, dass**
die aus dem Schaft (2) und dem Führungsrohr (8) gebildete kompakte Anordnung eine im Wesentlichen zylindrische Form aufweist.

14. Anordnung nach einem der vorhergehenden Ansprüche,
**dadurch gekennzeichnet, dass**
sie einen Teleskopmechanismus aufweist, der zumindest den Schaft (2) und eine Pistolenverlängerung aufweist.

15. Anordnung nach einem der vorhergehenden Ansprüche,
**dadurch gekennzeichnet, dass**
das Führungsrohr (8) eine geneigt verlaufende Vorderseite (40) aufweist.

16. Anordnung nach einem der vorhergehenden Ansprüche,
**dadurch gekennzeichnet, dass**
sie eine Einrichtung zum Erwärmen der Pistole (4) aufweist.

17. Anordnung nach einem der vorhergehenden Ansprüche,
**dadurch gekennzeichnet, dass**
die Sichtröhre (14) so ausgerichtet ist, dass eine Fokussierung des Bildes auf die Achse der vorderen Spitze des Arbeitswerkzeugs (4) in einem gewünschten Arbeitsabstand vor dem Führungsrohr (8) erzielt wird.

18. Anordnung nach einem der vorhergehenden Ansprüche,
**dadurch gekennzeichnet, dass**
die Sichtröhre (14) ein vorderes Ende aufweist, das so gebogen ist, dass eine Fokussierung des Bildes auf die Achse der vorderen Spitze des Arbeitswerkzeugs (4) in einem gewünschten Arbeitsabstand vor dem Führungsrohr (8) erzielt wird.

19. Anordnung nach einem der Ansprüche 1 bis 18,
**dadurch gekennzeichnet, dass**
sie ferner ein weiteres Arbeitswerkzeug (4) enthält, bei dem es sich um ein Werkzeug zum Abgeben von Medikamenten handelt.

20. Anordnung nach einem der Ansprüche 1 bis 18,
**dadurch gekennzeichnet, dass**
sie ferner ein weiteres Arbeitswerkzeug (4) enthält, bei dem es sich um ein Werkzeug zum Abhören von Tönen handelt.

21. Anordnung nach einem der Ansprüche 1 bis 18,
**dadurch gekennzeichnet, dass**
sie ferner ein weiteres Arbeitswerkzeug (4) enthält, bei dem es sich um ein Werkzeug zum Aufnehmen von Fotos handelt.

22. Anordnung nach einem der Ansprüche 1 bis 18,
**dadurch gekennzeichnet, dass**
sie ferner ein weiteres Arbeitswerkzeug (4) enthält, bei dem es sich um ein Werkzeug zur Entnahme von inneren Substanzen handelt.

## Claims

1. - A work assembly comprising:
- an apparatus for aiding in vaginal penetration comprising: a handling shaft (2); a guide tube (8) extending the handling shaft (2) to form a compact assembly, said guide tube (8) having a front end configured to be disposed before the cervix of an animal; and a video viewing tube of endoscope type (14), comprising lighting at its front end, the video viewing tube being received in the guide tube (8); the handling shaft (2) comprising a video system rear cell having means for transmitting, to a remote screen, images coming from the end of the video viewing tube; and
- a work tool, received in said apparatus, passing through the handling shaft, in relation to which it slides, whereby the work tool is movable between a position in which its front tip is received in the guide tube (8) and a position in which its front tip forward of the guide tube, said work tool being an insemination gun (4) configured to penetrate the vagina of said animal, reach said cervix of the uterus, pass the entrance and inject into the uterus of said animal the liquid semen contained in a straw or in a capsule.

2. - An assembly according to claim 1, **characterized in that** the front end of the viewing tube (14) is configured so as to achieve lighting and forward vision at the same time.

3. - An assembly according to any one of the preceding claims, **characterized in that** the viewing tube (14) comprises a video system with lighting of videoscopic or fibroscopic type.

4. - An assembly according to any one of the preceding claims, **characterized in that** the guide tube (8) comprises internally, next to the viewing tube (14), a tube (12) configured to receive the front tip of the work tool disposed therein.

5. - An assembly according to any one of the preceding claims, **characterized in that** the image transmission means comprise a connector for a connecting wire.

6. - An assembly according to claim 5, **characterized in that** said connector is of "USB" type.

7. - An assembly according to any one of claims 1 to 4, **characterized in that** the image transmission means comprise a wireless telecommunication means.

8. - An assembly according to any one of the preceding claims, **characterized in that** the rear cell of the apparatus is configured to receive electrical energy by an external cable connected to a tablet or a smartphone performing the display.

9. - An assembly according to any one of claims 1 to 7, **characterized in that** the rear cell of the apparatus comprises a battery incorporated into the handling shaft (2).

10. - An assembly according to any one of the preceding claims, **characterized in that** the guide tube (8) is made from an easily washable transparent material.

11. - An assembly according to any one of the preceding claims, **characterized in that** the guide tube (8), the viewing means (14), and a tube receiving the front tip of the work tool (12) disposed therein, are fastened to the handling shaft (2) by a mounting of bayonet type.

12. - An assembly according to any one of the preceding claims, **characterized in that** the guide tube (8) comprises at its base by the handling shaft (2), openings to the outside (42).

13. - An assembly according to any one of the preceding claims, **characterized in that** the compact assembly formed by the handling shaft (2) and the guide tube (8), comprise a generally cylindrical shape.

14. - An assembly according to any one of the preceding claims, **characterized in that** it comprises a telescopic mechanism comprising at least the handling shaft (2) and a gun extension.

15. - An assembly according to any one of the preceding claims, **characterized in that** the guide tube (8) comprises a front face (40) which is inclined.

16. - An assembly according to any one of the preceding claims, **characterized in that** it comprises a heating means of the gun (4).

17. - An assembly according to any one of the preceding claims, **characterized in that** the viewing tube (14) is oriented so as to obtain focusing of the image on the axis of the front tip of the work tool (4), at a desired work distance in front of the guide tube (8).

18. - An assembly according to any one of the preceding claims, **characterized in that** the viewing tube (14) comprises a front end bent so as to obtain focusing of the image on the axis of the front tip of the work tool (4), at a desired work distance in front of the guide tube (8).

19. - An assembly according to any one of claims 1 to 18, **characterized in that** it also comprises another work tool (4) which is a tool for depositing drugs.

20. - An assembly according to any one of claims 1 to 18, **characterized in that** it also comprises another work tool (4) which is a tool for listening to sounds.

21. - An assembly according to any one of claims 1 to 18, **characterized in that** it also comprises another work tool (4) which is a tool for taking photographs.

22. - An assembly according to any one of claims 1 to 18, **characterized in that** it also comprises another work tool (4) which is a tool for sampling internal substances.
